# EUROPEAN PATENT SPECIFICATION

(11) **EP 3 903 836 B1**
(45) Date of publication and mention of the grant of the patent: **09.04.2025**
(21) Application number: 21170658.5
(22) Date of filing: 27.04.2021
(51) Int. Cl.: A61L 2/08, A61L 2/10, A61L 2/24

(54) **A METHOD AND A SYSTEM OF DISINFECTING A ROOM**
VERFAHREN UND SYSTEM ZUR DESINFEKTION EINES RAUMES
PROCÉDÉ ET SYSTÈME DE DÉSINFECTION D'UNE PIÈCE

(30) Priority: 27.04.2020 US 202063016009 P
(43) Date of publication of application: 03.11.2021
(60) Divisional application: 22164016.2
(73) Proprietor: Carnegie Robotics, LLC, Pittsburgh, Pennsylvania 15201 (US)
(72) Inventor: LAROSE, David, Pittsburgh, PA 15201 (US); THYBO, Claus, 8230 Aabyhøj (DK)
(74) Representative: Inspicos P/S

(56) References cited:
- WO-A1-2015/116876
- WO-A1-2017/020028
- WO-A1-2018/089288
- US-A1- 2018 193 502
- US-A1- 2020 085 983

## Description

The present invention relates to a method and a system of disinfecting a room, such as a hospital room, operating theatre, doctors consultancy, nursery, nursing home or any other type of room.

In a first aspect, the invention relates to a method of disinfecting a room in accordance with claim 1.

Relevant technology may be seen in WO2018/089288, US2020/085983, WO2015/11676, US2018/193502 and WO2017/020028.

In the present context, disinfection is the killing of at least a percentage of germs, bacteria or viruses present on surfaces of the room. Disinfection may be performed in many manners and will have different effects on different germs, bacteria and viruses.

Disinfection may be obtained by launching disinfecting radiation, such as X-ray or UV, such as UV-C radiation, on to the surface. According to the invention, the disinfection is performed by launching radiation on to the surfaces. This has the advantage that the disinfection may be swift and may immediately leave the room in a condition where it may be inhabited.

A room may have any size and any surfaces. Often, rooms have therein furniture or other objects which may also need disinfection. Typical types of rooms are rooms at hospitals, doctors, clinics, nursing homes, and the like.

An irradiating unit is a unit configured to emit radiation or particles. The unit may comprise a larger area for outputting the radiation/particles. Thus, particles/radiation may be emitted from both a lower position and a higher position so that e.g. a bed may be disinfected both from below and above without the irradiating unit moving. Preferably, the radiation may reach no less than 1.5m, such as 2m from the floor of all surfaces in the room. In addition, it is preferred that radiation may be launched upwardly from a height of less than 50cm from the floor, such as less than 40cm. In addition, it may be desired that radiation/particles may be launched in a downward direction on to surfaces having a height of 1m from the floor. Thus a stationary irradiating unit preferably is able to emit radiation from at least two positions. In one embodiment, the irradiating unit comprises one or more elongate irradiating elements, such as fluorescent lamps, which are positioned vertically and which are configured to output radiation from a portion which is closer than 50cm to the floor and another portion which is above 100cm above the floor.

Clearly, as will be described further below, another embodiment has a movable arm with the radiation emitter, so that the emitter may be moved about in the room. An arm may be brought down to a height from which it may emit radiation upwardly from a sufficiently low position and later brought to a higher position from which it may launch radiation downwardly to a higher surface.

The irradiating unit is moved to a first position in the room from which radiation is emitted. While outputting the radiation, an intensity of the output radiation is determined. It is known that radiation emitters may change over time so that over time less radiation is output. This may be monitored using the intensity determination. When disinfecting a surface, it often is desired that a minimum dose of the radiation is launched on the surface in order to achieve a minimum disinfection. The dose is determined from the intensity directed toward the surface and the period of time during which the intensity is delivered. The intensity delivered to the surface is determined from the intensity output from the emitter and the distance to the surface, as well as the angle between the radiation and the surface.

The intensity of the radiation emitted may be the intensity close to the radiation emitter and may be determined by an element forming part of the irradiating unit. Radiation emitters exist which have built-in intensity monitors.

From the position and the intensity, it may be determined which intensity or dose has reached different surfaces, so that the disinfection may be quantified or estimated.

Further below, a unit capable of forming a 3D rendering or map of the room is described. This unit makes it possible to not only determine the intensity reaching individual surfaces but also surfaces which cannot be reached as they are provided behind elements forming shadows.

In one embodiment, the moving step comprises determining the first position. This determination may be a determination of the position in the room. The irradiating unit may comprise an element configured to determine in which position it is. The position determining element may be configured to determine its position from distances to e.g. the walls of the room and based on a map of the room. Alternatively, the position may be determined in advance and the unit positioned at the position.

The position may be identified as a coordinate in a map of the room, as may furniture and other elements or items in the room, such as equipment, luggage and other elements. Especially interesting are items which may generate shadows in the room so that some surface portions are not reached by the radiation. The position may be in a horizontal plane, such as projected on to the floor of the room. In addition, it may be desired to know the 3D position and direction of surfaces and elements in the room so that the intensity of the radiation impinging on the surfaces may be determined. From this, also the emission characteristics of the irradiating unit are desired as mentioned above.

As indicated above, a period of time is determined during which the intensity was emitted. This may be used for determining a resulting dose of radiation delivered to a surface portion.

In one embodiment, the outputting step comprises comparing the first position with a predetermined position and outputting any discrepancy between the first and predetermined positions. If a discrepancy or offset is seen, the irradiation may not be as expected. Then, the irradiating unit will be closer to some surfaces, thus delivering more radiation to these, but farther from other surfaces which then will receive less radiation. Thus, an insufficient disinfection may be obtained.

Also, it may be desired to not irradiate some surfaces more than necessary. Disinfecting radiation may have detrimental effect on some materials, such as some polymers, which age more rapidly when irradiated. Thus, not only a lower radiation may be problematic but also a non-desired excessive irradiation may be seen by the offset.

The offset may thus be counteracted at least for the insufficient irradiation by irradiating the pertaining surfaces more at a later point in time.

Naturally, also discrepancies or deviations from a predetermined intensity may cause problems, especially if the intensity output is lower than expected. This may also be reported, such as when the outputting step comprises comparing the determined intensity with a predetermined intensity and outputting any discrepancy between the determined and predetermined intensities. In addition or alternatively, a discrepancy in the time of irradiation may be logged, as also this may lead to a discrepancy of the dose delivered to e.g. a surface part.

In general, the information output may serve to either indicate that a disinfection plan is observed or that deviations have been seen from this plan. From such deviations, the impact may be determined, such as whether surfaces have been sufficiently disinfected or whether further disinfection is desired. Preferably, the plan would comprise positions and intensities, and optionally also periods of time of the irradiation, so that when following the plan, all relevant surface parts receive sufficient irradiation.

Further below, a unit and a method is described which would be well suited for use in this method. This unit and method is capable of not only irradiating the room but also to intelligently do so.

A second aspect of the invention relates to a system according to claim 7.

In the present context, a system is an assembly of elements which may be fixed to each other but which may be separate and in communication with each other. Communication may be wireless or wired and performed on any desired protocol.

A self-propelled unit is a unit which may move on its own. The unit thus may have a motor or other actuator capable of moving the unit. The unit may have a power source, such as a battery, fuel cell or solar panels, or may be powered by a wire from e.g. mains in the room.

More intelligent units may be robot-like units which are able to sense their surroundings so as to be able to manoeuvre independently, such as based on a map of the room. Then, the unit may itself move to the desired position.

An irradiating unit is a unit capable of outputting disinfecting radiation, ions or particles. Preferably, the irradiating unit is capable of emitting radiation. Then, the intensity output may be controlled and determined as may the emission characteristics, such as a wavelength/energy of the radiation, the intensity distribution and the like.

The irradiating unit may have an emission distribution capable of irradiating both high and low surfaces and around an angle of at least 100 degrees, such as at least 200 degrees, such as at least 300 degrees around a vertical axis. In one embodiment, the irradiating unit comprises one or more elongate fluorescent lamps capable of irradiating 360 degrees around a vertical axis. In another embodiment, the irradiating unit is positioned on a movable arm capable of being directed in all directions so as to be able to irradiate surfaces with any direction.

The position determining element may be an element capable of driving the self-propelled unit to the position or an element configured to determine a position in which the self-propelled unit is. The position may be a coordinate or other indication which may be entered by a user/operator or the like.

A controller may be any type of processor, ASIC, FPGA, DSP, microprocessor, hardwired or software controlled. The controller may be a combination of several such elements configured to communicate via any protocol and on any medium. Clearly, part of the controller and/or an operation thereof may be handled by a remote server and/or a cloud solution.

The controller is configured to determine the intensity. This intensity may be reported from the irradiating unit or from a sensor positioned so as to receive radiation from the irradiating unit. The intensity may alternatively be determined from a power consumption of the irradiating unit.

The controller is configured to output information relating to the position and the determined intensity or dose. As described above, this information may be used for determining or estimating the disinfection of the room, such as of different surface or surface parts of the room.

The intensity determining element is also configured to determine a period of time during which the intensity was emitted.

In one embodiment, the controller is also configured to compare the first position with a predetermined position and output any discrepancy between the first and predetermined positions. The positions may be identified by coordinates or the like. A map of the room may be determined in which the positions may be indicated. The discrepancy may be a distance and/or a direction. Often, the discrepancy is determined in a horizontal plane, but in some cases, the discrepancy may be determined even in three dimensions.

In one embodiment, the controller is configured to compare the determined intensity with a predetermined intensity and output any discrepancy between the determined and predetermined intensities. As described above, this information may be used for outputting information from which the degree of disinfection may be determined. Also, compliance with a disinfection plan may be determined from the discrepancies.

An interesting technology relates to a method of disinfecting a room, the method comprising the steps of:
- providing a 3D rendering or map of the room,
- determining a plurality of positions from which radiation may disinfect predetermined surfaces of the room, and
- controlling a self-propelled unit to sequentially move to the positions and emit disinfecting radiation.

Clearly, all aspects, embodiments and situations of the invention may be interchanged. The present technology may easily be combined with the method of the first aspect of the invention, for example.

In the present context, providing a 3D rendering or map of the room may be the receiving of the rendering or map. A 3D rendering or map is information from which the positions or relative positions of elements, such as furniture, equipment and the like, may be determined. This rendering or map may be used for navigating in the room. Alternatively or additionally, the rendering or map may be used for determining positions and angles of surfaces. This may be relevant for determining a dose delivered from an irradiating element positioned in a predetermined position in the room. The 3D rendering or map may be generated by usual means such as radar, lidar, sonar, stereo imaging, laser scanning, structure from motion, and the like. Structured light may be launched into the room and the reflected radiation may be used for determining the 3D positions of surfaces in the room.

According to the method, a plurality of positions are determined from which radiation may disinfect predetermined surfaces of the room. A position may be that of an irradiating unit. In one embodiment, as described above, the irradiating unit may have stationary irradiating elements mounted to it. In other embodiments, the irradiating unit may comprise a movable arm from which the radiation is emitted.

The position is that from which the radiation is emitted. Thus, if the radiation emitter is stationary on the unit, the position may be that of the unit. If the radiation is emitted from an arm, the position may be that of the unit or of the arm.

The advantage of providing multiple positions is that any element in the room may generate a shadow shielding surface parts from receiving the radiation and thus be disinfected. Thus, when disinfecting by irradiating from one position, some surface portions may not receive radiation or not receive sufficient radiation. In some situations, some elements in the room will be over-exposed before other elements have received sufficient radiation. Then, another position may be determined from which sufficient radiation may be received by surfaces which have not yet received enough radiation.

Thus, the room may have a first surface which is not visible from a first position but is visible from the second position and a second surface not visible from the second position but is visible from the first position.

Then, a self-propelled unit is controlled to sequentially move to the positions and emit disinfecting radiation. In this manner, the intensity or dose delivered to one or more surface parts may be determined so that information may be generated as to the degree of disinfection of the surface part. This is described above.

In one embodiment, the providing step is performed before the controlling step. Thus, the rendering or map is first generated. This may be generated in advance, such as by another unit, or the room may be standardized so that a predetermined map or rendering may be used over time. Alternatively, the self-propelled unit may comprise mapping elements, such as radar, lidar or other elements as described above and below, and may derive the map or rendering before determining the positions to visit. Generating a rendering or map of a room is a standard technology.

Alternatively, the unit may enter the room and perform the irradiation before or while performing at least part of the rendering or mapping. Then, from that information, the unit may determine a next position from which irradiation is then performed while, potentially, further mapping/rendering may be performed. This process may be repeated until a desired disinfection is performed of all relevant surfaces.

Even from the first rendering/mapping, potentially shadowing elements may be identified, such as from sudden distance changes. From a rendering or map made from one position, a TV, for example, will be seen as an element having a certain distance from the wall. Thus, it may be inferred that the TV will generate a shadow in which a wall portion is positioned.

In one embodiment, as described above, the step of emitting the radiation comprises emitting the radiation from an element movable vis-à-vis a base of the unit. In this situation, the movement of the arm is preferably tracked while outputting the radiation in order to determine which surfaces are irradiated and with which intensity.

In another embodiment, as described, the irradiating unit is stationary relative to the self-propelled unit.

In one embodiment, the method further comprises the step of outputting information relating to surfaces irradiated. In this manner, this information may be used for indicating the degree of disinfection of the room. Preferably, the information output further comprises information relating to a radiation dose received by one or more portions of the irradiated surfaces. This may indicate the degree of disinfection and may be used by the user or owner of the room for proving the degree of disinfection. This information may be so detailed that the degree of disinfection of individual surface portions may be determined.

Then, the outputting step may comprise comparing the information relating to the radiation dose to predetermined doses and outputting a result of the comparison. This may reveal surface portions having received insufficient radiation and which may the not be sufficiently disinfected.

As mentioned above, it may also be desired to not irradiate some surfaces or material excessively. In one embodiment, the providing step comprises identifying one or more surfaces of the room, and wherein the controlling step comprises determining, for each identified surface, a dose interval, and wherein the step of emitting the radiation comprises emitting radiation toward each identified surface to obtain a radiation dose on each surface within the interval for that surface.

Some materials may deteriorate too swiftly if irradiated excessively. It may be desired to limit the overall dose to a dose within an interval. Alternatively, it may be desired to limit the intensity delivered to an interval. In the latter situation, a desired dose may be obtained by limiting the intensity but increasing the irradiating time.

In this situation, the identification may be based on a shape or position of the surface. A particular element, such as a computer, a desk, a TV or the like may be identified. From predetermined knowledge, it may be known which dose or intensity interval that element or its surface is capable of handling.

Alternatively, the surface may be determined from e.g. an image (2D or 3D) thereof. A surface texture or structure may be estimated. Reflection of radiation may also be used for identifying a surface or the material thereof.

Some surfaces may need a lower intensity or dose, such as if bacteria or the like are not able to move into the material but must remain on the surface thereof.

Other materials may be more porous or open and may then require more intensity or radiation as the bacteria and the like may be provided inside this material and thus may be more or less shielded by the material. Alternatively, it may be desired to irradiate such surfaces from multiple angles so that the identifying step may comprise identifying a surface with predetermined characteristics, and wherein the emitting step comprises emitting radiation toward the pertaining surface from a plurality of angles.

A surface may be identified by providing relevant information, such as an image, to an operator which may enter the material or desired irradiating parameter which are then followed by the unit. Thus, in one embodiment, the step of identifying a surface comprises:
∘ providing 2D or 3D information of the surface,
∘ outputting the information to an operator and
∘ receiving identifying information from the operator.

In one embodiment, as mentioned above, the providing step comprises obtaining 3D information from the room using a radar, lidar, sonar, stereo vision set-up, structure from motion, or the like.

Yet another interesting technology is one relating to a system for disinfecting a room, the system comprising:
- a storage for holding a 3D rendering or map of the room,
- a self-propelled unit comprising a radiation emitter and
- a controller configured to:
   - determine a plurality of positions from which radiation may disinfect predetermined surfaces of the room,
   - control the self-propelled unit to sequentially move to the positions and emit disinfecting radiation.

The storage may be any type of storage, such as RAM, ROM, Flash or the like. The 3D rendering or map may be stored on any desired format.

As mentioned above, the rendering or map may be generated by the self-propelled unit or may be loaded into the storage from a remote system, such as a server. The rendering or map may be downloaded for each irradiation of each room or may be a standard rendering useful for more rooms or the same room multiple times.

Alternatively, the map or rendering may be generated by a separate unit which may enter the room independently of the self-propelled unit. The mapping or rendering unit may itself be self-propelled if desired.

The self-propelled unit may be as described above. The self-propelled unit comprises a radiation emitter which may emit the radiation or particles described above and which may have the structure and capabilities described above.

The controller may be as described above. The controller is configured to determine a plurality of positions from which radiation may disinfect predetermined surfaces of the room. As described above, the determination of the positions may be made for each irradiation of the room, such as if the setting of the room changes from time to time. Alternatively, the positions may have been determined previously and are re-used.

As mentioned above, the positions preferably aim at ensuring that all relevant surface parts are irradiated and disinfected.

Clearly, not all surface parts may need disinfection. It may not be desired or required to disinfect lamps, ceilings, the frame of beds/chairs/tables or the like. Alternatively, different disinfecting schemes may be defined, where a thorough disinfecting comprises disinfecting of more surface parts than a less thorough disinfection. It may be desired to perform the less thorough disinfection more often and then with a lower frequency the more thorough disinfection.

The controller is capable of controlling the self-propelled unit to sequentially move to the positions and emit disinfecting radiation. The controller then may be able to control one or more motors or actuators causing the unit to move. Also, the controller may be in communication with sensors and the like capable of determining at which position the unit is so that movement to the desired position is possible. The controller may also control the emission of the radiation, such as not only turning the radiation on/off but also controlling the intensity output. The radiation may not be homogeneous around the unit, so that rotation of the unit or the irradiating element may be desired to obtain the desired irradiation. If the unit comprises a radiation emitting arm, the controller may also control this.

In one embodiment, the controller is configured to operate the sensor to generate the 3D rendering or map and subsequently determine the positions and control the unit. Then, the unit may itself generate the map/rendering. In that manner, the unit may not need knowledge of the room before entering it to disinfect it.

As described above, the unit may choose to first provide the map or rendering and then determine the positions and perform the irradiation. Alternatively, the map/rendering may be generated iteratively while irradiation is performed from the individual positions.

As described, in one embodiment, the unit comprises a base unit and an element movable in relation to the base, the radiation emitter being provided in the movable element. In this situation, the controller may also be capable of operating this movable element.

In one embodiment, the controller is further configured to output information relating to surfaces irradiated. This information may be the position of the surfaces or the direction to the surfaces, such as from a predetermined point in the room. In addition or alternatively, the controller may be configured to further output information relating to a radiation dose received by one or more portions of the irradiated surfaces. In this manner, the degree of disinfection of individual surface portions may be determined.

In one embodiment, the controller is further configured to compare the information relating to the radiation dose to predetermined doses and output a result of the comparison. The predetermined doses may be desired minimum doses so that the comparison may identify surface portions which have not been sufficiently disinfected. Even though the unit is capable of moving between different positions in the room, it may not be possible to get sufficiently close to a surface to disinfect it properly without, for example, excessively irradiating other surfaces.

As described above, it may be possible to excessively irradiate some surfaces. Also, some surfaces or materials may require more irradiation than others. Then, in one embodiment, the controller is configured to:
- identify one or more surfaces of the room,
- determine, for each identified surface, a dose interval, and
- control the radiation emitter to emit the radiation comprises emitting radiation toward each identified surface to obtain a radiation dose on each surface within the interval for that surface.

In one embodiment, as mentioned above, the controller is configured to identify a surface with predetermined characteristics and control the emitter to emit radiation toward the pertaining surface from a plurality of angles.

Clearly, a disinfecting plan may then be defined comprising positions/paths and irradiation times, so that an optimal disinfection may be obtained. From knowledge of the surface parts, their position(s) and potentially other parameters, a disinfecting plan may be derived ensuring sufficient irradiation of all surface parts. If surface parts also have a maximum intensity or dose, this may be incorporated into the plan. Such plans may be arrived at using e.g. convex optimization or MLD (Mixed Logical Dynamics) methods.

In one embodiment, the controller is configured to identify a surface by:
o providing 2D or 3D information of the surface,
∘ outputting the information to an operator and
∘ receiving identifying information from the operator.

Having received the information from the operator, this information may be stored so that there is no need to ask the next time a surface with the same characteristics, such as colour, colour variation, texture, structure or the like, is seen.

In one embodiment, as described above, the apparatus further comprises a sensor configured to generate the 3D rendering or map.

In the following, preferred embodiments are described with reference to the drawing, wherein:
- Figure 1 illustrates a hospital room,
- Figure 2 illustrates a first embodiment of an irradiation robot,
- Figure 3 illustrates a second embodiment of an irradiation robot.

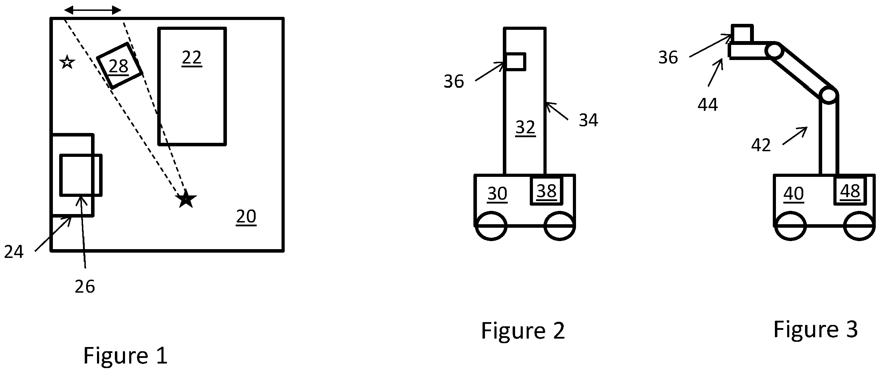

In figure 1, a hospital room 20 is illustrated with a bed 22, a table 24, a chair 26 and bedside equipment 28. It is desired to disinfect the room using disinfecting radiation, such as UV radiation.

Figure 2 illustrates a first embodiment of a robot 30 suitable for disinfecting a room. The robot has an upstanding portion 32 comprising one or more outwardly directed radiation emitters 34 configured to emit disinfecting radiation from the emitters and toward the surroundings, i.e. surfaces of the room and its contents.

A problem encountered using a radiation emitting robot is that shadows may occur, i.e. surfaces exist which may not be irradiated and thus disinfected. In figure 1, a fat star marks a radiation emitting robot positioned at the star. It is seen that the equipment 28 receives the radiation and thus prevents the radiation from reaching the wall behind it. A shadow is generated as indicated by the double arrow.

The solution may be to move the robot subsequently (or before) to the position of the slender star, as the wall portion may be disinfected from this position.

A more autonomous robot is desired, so the robot 30 comprises a sensor 36 configured to generate a 3D map or rendering of the room and the contents. Thus, the robot is configured to realize that the equipment 28 will generate a shadow and thus determine a path to take while irradiating or different positions from which the irradiation may be performed to reduce or eliminate the shadows.

It is noted that the position of shadows will depend also on from which height the radiation is emitted. Thus, it may be desired to emit radiation from different heights of the robot so that radiation may be emitted below the bed or table and from a direction further below so that the radiation is directed upwardly to surfaces below the bed and table, for example.

Naturally, generating a 3D rendering or map may require obtaining data from different heights in the room. This may be obtained by using sensors provided at different heights on the robot 30. It is desired to generate the information at least for all angles of attack of the radiation. Alternatively, the sensor 36 may be translatable up and down along the portion 32 to provide the information needed to generate the map/rendering. Also, it may be desired that the sensor may then be moved away from the radiation emitter so as to not itself create shadows.

Figure 3 illustrates another embodiment of a robot 40 configured to emit disinfecting radiation. The robot 40 comprises an arm 42 with a head portion comprising radiation emitter(s) 44. The arm may be moved in any desired manner to direct the radiation toward the surfaces, such as on the bed, below the bed, on the walls and the like. The use of the arm makes it easier to reduce the size of the shadows even when the robot is stationary. However, shadows may still exist, and the robot 40 also comprises the sensor 36 for generating the 3D map or rendering.

The sensor 36 may be based on stereo vision, laser scanning, sonar, radar, lidar, or any other desired rendering technology. The sensor 36 may also comprise a controller configured to interpret the sensed radiation, sound or the like to arrive at the 3D map or rendering. Alternatively, a more central controller 38/48 may be used which can also be used for controlling the robot, such as the movement thereof, the operation of the radiation emitter(s) as well as the arm if present.

In general, the mapping/rendering sensor 36 may be provided on a separate arm if desired so that its movement may be independent of the movement of the irradiating element.

The sensor 36 may be used for additional purposes. Having provided the 3D rendering/map, the surfaces of the room are determined. The sensor, controller or robot may now determine the dose of the disinfecting radiation received by each surface or portion of each surface. Thus, it may be ensured and even proved that each surface has received sufficient radiation to be disinfected. The dose received depends on the intensity emitted toward the surface portion, the distance to the surface portion as well as the period of time of the irradiation. Also, the angle between the general direction of the wall portion and that of the radiation should be taken into account. This information is available to the robot, so that the radiation intensity and/or the irradiation time may be controlled to arrive at the desired disinfection. Additionally, this information may be logged for later proof that the room was sufficiently disinfected.

In fact, information may be derived from the map/rendering as to what the surfaces may be formed by. For example, from reflection or surface texture, it may be determined or assumed that a surface is steel, hard plastics, soft plastics/rubber, wood, glass, textile or the like. Alternatively, a standard 2D image may be used from which colours, surface texture and the like may be estimated. From this, the desired dose may be determined. Steel, glass and hard plastics may be assumed to have a hard and non-porous surface and thus need less radiation than wood or textile which may have a porosity in which bacteria and/or virus particles may settle. Such porous surfaces may require a higher dose to be disinfected. Thus, in addition to tracking the dose delivered to different surface parts, also the type of surface part may be tracked and logged to determine the dose required and in order to also use this information to prove that the room is disinfected.

The surface may also be identified from its shape, appearance and/or position. A TV may be mounted on the wall and a computer may be identified by its shape. From that information, it may be decided how to irradiate them. The controller may hold predetermined information as to how to irradiate such elements. For example a keyboard of a computer may be especially contaminated whereas it may be desired to irradiate a monitor less intensely. Also, elements such as keyboards may require irradiation from multiple sides due to porosity/grooves or the like therein.

If a surface cannot be identified by the controller, information, such as an image may be output to an operator, which may feedback information of the material of the surface and/or how to irradiate the surface, such as minimum dose, maximum intensity, multiple angles or the like.

Other parameters to take into account may be a maximum intensity which a material or surface may be able to withstand. It may be desired to deliver a dose to some materials or surfaces over a longer period of time. Some plastic materials, for example age faster at higher intensities.

Alternatively, an upper dose limit may be set for surface portions. This may also be based on the type or material forming the surface.

Then, the robot may then start by generating the 3D map/rendering or a part thereof and determine a position from which to start irradiating. Using the robot 40, the radiation may be controlled rather precisely. With the robot 30, multiple positions may be determined and used in order to not only avoid the shadows but also to control the distance to surface portions and thus the dose delivered.

Clearly, the robot(s) may be able to control the emission of radiation. Thus, the intensity may be controlled as well as the angle of emission. The robot 30 may be able to emit radiation 360 around a vertical axis through the portion 32 but may be able to control the intensity emitted in some or all directions in order to tailor the dose delivered to different surface portions.

Having finished the radiation at one position, the robot may move to another position, which may be determined from the 3D map/rendering, where after irradiation is resumed. This may be repeated until a sufficient irradiation of all relevant surface portions of the room have taken place.

As mentioned, the irradiation may be logged so that information may be retrieved relating to the dose delivered to different surface portions. Also, this information may relate to the surface portion, such as an assumed material, porosity or the like.

## Claims

1. A method of disinfecting a room comprising a surface, the method comprising:
- moving an irradiating unit to a first position in the room,
- emitting radiation from the irradiating unit toward the surface, during a period of time, while determining an intensity of emitted radiation emitted to the surface,
- determining a dose of radiation launched on the surface from the determined intensity and the period of time,
- outputting information relating to the position and the determined dose,
**characterized in that** the intensity delivered to the surface is determined from an intensity output from the emitter, a distance to the surface, and an angle between the radiation and the surface.

2. A method according to claim 1, wherein the moving step comprises determining the first position.

3. A method according to any of the preceding claims, wherein the outputting step comprises comparing the first position with a predetermined position and outputting any discrepancy between the first and predetermined positions.

4. A method according to any of the preceding claims, wherein the outputting step comprises comparing the determined intensity with a predetermined intensity and outputting any discrepancy between the determined and predetermined intensities.

5. A method according to any of the preceding claims, wherein the dose is determined also based on a distance to the surface.

6. A system for disinfecting a room comprising a surface, the system comprising
- a self-propelled unit comprising
- an irradiating unit,
- an intensity determining element and
- a position determining element and
- a controller configured to:
- determine an intensity of emitted radiation launched toward the surface and a period of time during which the radiation was launched toward the surface, and
- output information relating to the position and a dose of radiation delivered to the surface based on the intensity and the period of time,
**characterized in that** the controller is configured to determine the intensity delivered to the surface from an intensity output from the emitter, a distance to the surface, and an angle between the radiation and the surface.

7. A system according to claim 6, wherein the controller is also configured to compare the first position with a predetermined position and output any discrepancy between the first and predetermined positions.

8. A system according to any of claims 6-7, wherein the controller is configured to compare the determined intensity with a predetermined intensity and output any discrepancy between the determined and predetermined intensities.

9. A system according to any of claims 6-8, wherein the controller is configured to determine the dose also based on a distance to the surface.

## Patentansprüche

1. Verfahren zum Desinfizieren eines Raums, umfassend eine Oberfläche, wobei das Verfahren Folgendes umfasst:
- Bewegen einer Bestrahlungseinheit an eine erste Position in dem Raum,
- Emittieren von Strahlung von der Bestrahlungseinheit zu der Oberfläche während eines Zeitraums, während eine Intensität von emittierter Strahlung, die auf die Oberfläche emittiert wird, bestimmt wird,
- Bestimmen einer Strahlungsdosis, die auf die Oberfläche abgeschossen wird, anhand der bestimmten Intensität und des Zeitraums,
- Ausgeben von Informationen in Bezug auf die Position und die bestimmte Dosis,
**dadurch gekennzeichnet, dass** die Intensität, die an die Oberfläche abgegeben wird, anhand einer Intensitätsausgabe von dem Emitter, einem Abstand zu der Oberfläche und einem Winkel zwischen der Strahlung und der Oberfläche bestimmt wird.

2. Verfahren nach Anspruch 1, wobei der Bewegungsschritt Bestimmen der ersten Position umfasst.

3. Verfahren nach einem der vorhergehenden Ansprüche, wobei der Ausgabeschritt Vergleichen der ersten Position mit einer vorbestimmten Position und Ausgeben jeglicher Abweichung zwischen der ersten und der vorbestimmten Position umfasst.

4. Verfahren nach einem der vorhergehenden Ansprüche, wobei der Ausgabeschritt Vergleichen der bestimmten Intensität mit einer vorbestimmten Intensität und Ausgeben jeglicher Abweichung zwischen der bestimmten und der vorbestimmten Intensität umfasst.

5. Verfahren nach einem der vorhergehenden Ansprüche, wobei die Dosis ferner basierend auf einem Abstand zu der Oberfläche bestimmt wird.

6. System zum Desinfizieren eines Raums, umfassend eine Oberfläche, wobei das System Folgendes umfasst:
- eine selbstangetriebene Einheit, umfassend
- eine Bestrahlungseinheit,
- ein Intensitätsbestimmungselement und
- ein Positionsbestimmungselement und
- eine Steuerung, die zu Folgendem konfiguriert ist:
- Bestimmen einer Intensität von emittierter Strahlung, die auf die Oberfläche abgeschossen wird, und eines Zeitraums, während dem die Strahlung auf die Oberfläche abgeschossen wurde, und
- Ausgeben von Informationen in Bezug auf die Position und eine Strahlungsdosis, die an die Oberfläche abgegeben wird, basierend auf der Intensität und dem Zeitraum,
**dadurch gekennzeichnet, dass** die Steuerung dazu konfiguriert ist, die Intensität, die auf die Oberfläche abgegeben wird, anhand einer Intensitätsausgabe von dem Emitter, einem Abstand zu der Oberfläche und einem Winkel zwischen der Strahlung und der Oberfläche zu bestimmen.

7. System nach Anspruch 6, wobei die Steuerung ferner dazu konfiguriert ist, die erste Position mit einer vorbestimmten Position zu vergleichen und jegliche Abweichung zwischen der ersten und der vorbestimmten Position auszugeben.

8. System nach einem der Ansprüche 6-7, wobei die Steuerung dazu konfiguriert ist, die bestimmte Intensität mit einer vorbestimmten Intensität zu vergleichen und jegliche Abweichung zwischen der bestimmten und der vorbestimmten Intensität auszugeben.

9. System nach einem der Ansprüche 6-8, wobei die Steuerung dazu konfiguriert ist, die Dosis ferner basierend auf einem Abstand zu der Oberfläche zu bestimmen.

## Revendications

1. Procédé de désinfection d'une pièce comprenant une surface, le procédé comprenant **:**
- le déplacement d'une unité d'irradiation vers une première position dans la pièce,
- l'émission d'un rayonnement depuis l'unité d'irradiation vers la surface, pendant une période de temps, tout en déterminant une intensité de rayonnement émis vers la surface,
- la détermination d'une dose de rayonnement lancée sur la surface à partir de l'intensité déterminée et de la durée,
- la délivrance en sortie d'informations relatives à la position et à la dose déterminée,
**caractérisé en ce que** l'intensité délivrée à la surface est déterminée à partir d'une intensité délivrée en sortie de l'émetteur, d'une distance à la surface et d'un angle entre le rayonnement et la surface.

2. Procédé selon la revendication **1,** dans lequel l'étape de déplacement comprend la détermination de la première position.

3. Procédé selon l'une quelconque des revendications précédentes, dans lequel l'étape de délivrance en sortie comprend la comparaison de la première position avec une position prédéterminée et la délivrance en sortie de toute différence entre la première position et la position prédéterminée.

4. Procédé selon l'une quelconque des revendications précédentes, dans lequel l'étape de délivrance en sortie comprend la comparaison de l'intensité déterminée avec une intensité prédéterminée et la délivrance en sortie de toute différence entre les intensités déterminées et prédéterminées.

5. Procédé selon l'une quelconque des revendications précédentes, dans lequel la dose est déterminée également sur la base d'une distance à la surface.

6. Système de désinfection d'une pièce comprenant une surface, le système comprenant
- une unité automotrice comprenant
- une unité d'irradiation,
- un élément déterminant l'intensité et
- un élément déterminant la position et
- un contrôleur configuré pour :
- déterminer une intensité de rayonnement émis lancé vers la surface et une période de temps pendant laquelle le rayonnement a été lancé vers la surface, et
- délivrer en sortie des informations relatives à la position et à une dose de rayonnement délivrée à la surface sur la base de l'intensité et de la durée,
**caractérisé en ce que** le contrôleur est configuré pour déterminer l'intensité délivrée à la surface à partir d'une d'intensité délivrée en sortie de l'émetteur, d'une distance à la surface et d'un angle entre le rayonnement et la surface.

7. Système selon la revendication 6, dans lequel le contrôleur est également configuré pour comparer la première position avec une position prédéterminée et générer toute différence entre la première position et la position prédéterminée.

8. Système selon l'une quelconque des revendications 6 à 7, dans lequel le contrôleur est configuré pour comparer l'intensité déterminée avec une intensité prédéterminée et générer toute différence entre l'intensité déterminée et l'intensité prédéterminée.

9. Système selon l'une quelconque des revendications 6 à 8, dans lequel le contrôleur est configuré pour déterminer la dose également sur la base d'une distance à la surface.
